# EUROPEAN PATENT SPECIFICATION

(11) **EP 2 966 983 B1**
(45) Date of publication and mention of the grant of the patent: **28.07.2021**
(21) Application number: 14718749.6
(22) Date of filing: 13.03.2014
(51) Int. Cl.: A01H 1/06, A01H 5/10, C12N 9/22, C12N 15/82

(54) **MODIFYING SOYBEAN OIL COMPOSITION THROUGH TARGETED KNOCKOUT OF THE FAD2-1A/1B GENES**
VERÄNDERUNG DER ÖLZUSAMMENSETZUNG VON SOJABOHNEN DURCH GEZIELTE AUSSCHALTUNG DER FAD2-1A/1B GENE
MODIFICATION DE LA COMPOSITION DE L'HUILE DE SOJA PAR INACTIVATION CIBLÉE DES GÈNES FAD2-1A/1B

(30) Priority: 15.03.2013 US 201361790655 P; 01.10.2013 US 201361885213 P
(43) Date of publication of application: 20.01.2016
(73) Proprietor: Cellectis, 75013 Paris (FR)
(72) Inventor: MATHIS, Luc, F-94270 Le Kremlin Bicetre (FR); VOYTAS, Daniel F., Falcon Heights, Minnesota 55108 (US); ZHANG, Feng, Maple Grove, MN 55311 (US); HAUN, William, St. Paul, MN 55117 (US)
(74) Representative: Fish & Richardson P.C.
(86) International application number: PCT/IB2014/059752
(87) International publication number: WO 2014/141147

(56) References cited:
- WO-A1-2011/049627
- ANH-TUNG PHAM ET AL: "A novel-allele in a soybean plant introduction offers an alternate means to produce soybean seed oil with 85% oleic acid content", THEORETICAL AND APPLIED GENETICS ; INTERNATIONAL JOURNAL OF PLANT BREEDING RESEARCH, SPRINGER, BERLIN, DE, vol. 123, no. 5, 17 June 2011 (2011-06-17) , pages 793-802, XP019939043, ISSN: 1432-2242, DOI: 10.1007/S00122-011-1627-3
- PHAM ANH-TUNG ET AL: "Mutant alleles of FAD2-1A and FAD2-1B combine to produce soybeans with the high oleic acid seed oil trait", BMC PLANT BIOLOGY, BIOMED CENTRAL, LONDON, GB, vol. 10, no. 1, 9 September 2010 (2010-09-09), page 195, XP021073708, ISSN: 1471-2229, DOI: 10.1186/1471-2229-10-195 cited in the application
- TOMAS CERMAK ET AL: "Efficient design and assembly of custom TALEN and other TAL effector-based constructs for DNA targeting", NUCLEIC ACIDS RESEARCH, OXFORD UNIVERSITY PRESS, GB, vol. 39, no. 12, 1 July 2011 (2011-07-01), pages 1-11, XP002659530, ISSN: 1362-4962, DOI: 10.1093/NAR/GKR218 [retrieved on 2011-04-14]
- MAGDY M. MAHFOUZ ET AL: "TALE nucleases and next generation GM crops", GM CROPS, vol. 2, no. 2, 1 April 2011 (2011-04-01), pages 99-103, XP055093375, ISSN: 1938-1999, DOI: 10.4161/gmcr.2.2.17254
- S. J. CURTIN ET AL: "Targeted Mutagenesis of Duplicated Genes in Soybean with Zinc-Finger Nucleases", PLANT PHYSIOLOGY, vol. 156, no. 2, 4 April 2011 (2011-04-04) , pages 466-473, XP055130125, ISSN: 0032-0889, DOI: 10.1104/pp.111.172981 cited in the application -& Shaun J Curtin ET AL: "Text S1. Supplemental Methods Hairy root transformation of soybean cotyledons", Plant Physiology, Vol. 156 (2), 4 April 2011 (2011-04-04), XP055130151, DOI: 10.1104/pp.111.172981 Retrieved from the Internet: URL:http://www.plantphysiol.org/content/su ppl/2011/04/04/pp.111.172981.DC2/172981Sup plemental_Methods.pdf [retrieved on 2014-07-18]
- WILLIAM HAUN ET AL: "Improved soybean oil quality by targeted mutagenesis of the fatty acid desaturase 2 gene family", PLANT BIOTECHNOLOGY JOURNAL, vol. 12, no. 7, 23 May 2014 (2014-05-23), pages 934-940, XP055358281, GB ISSN: 1467-7644, DOI: 10.1111/pbi.12201

### SEQUENCE LISTING

<110> Cellectis
<120> MODIFYING SOYBEAN OIL COMPOSITION
   THROUGH TARGETED KNOCKOUT OF THE FAD2-1A/1B GENES
<130> 36861-0002WO1
<150> 61/885,213
   <151> 2013-10-01
<150> 61/790, 655
   <151> 2013-03-15
<160> 39
<170> FastSEQ for Windows Version 4.0
<210> 1
   <211> 289
   <212> DNA
   <213> Glycine max
<400> 1
   ttcattttct acattgccac cacctacttc cacctccttc ctcaaccctt ttccctcatt 60
   gcatggccaa tctattgggt tctccaaggt tgccttctca ctggtgtgtg ggtgattgct 120
   cacgagtgtg gtcaccatgc cttcagcaag taccaatggg ttgatgatgt tgtgggtttg 180
   acccttcact caacactttt agtcccttat ttctcatgga aaataagcca tcgccgccat 240
   cactccaaca caggttccct tgaccgtgat gaagtgtttg tcccaaaac              289
<210> 2
   <211> 289
   <212> DNA
   <213> Glycine max
<400> 2
   ttcattttct acattgccac cacctacttc cacctcctcc ctcacccctt ttccctcatt 60
   gcatggccaa tctattgggt tctccaaggt tgcattctta ctggcgtgtg ggtgattgct 120
   cacgagtgtg gtcaccatgc cttcagcaag tacccatggg ttgatgatgt tatgggtttg 180
   accgttcact cagcactttt agtcccttat ttctcatgga aaataagcca tcgccgccac 240
   cactccaaca cgggttccct tgaccgtgat gaagtgtttg tcccaaaac              289
<210> 3
   <211> 76
   <212> DNA
   <213> Glycine max
<400> 3
   gccaccacct acttccacct ccttcctcaa cccttttccc tcattgcatg gccaatctat 60
   tgggttctcc aaggtt 76
<210> 4
   <211> 60
   <212> DNA
   <213> Artificial Sequence
<220>
   <223> synthetic oligonucleotide
<400> 4
   gccaccacct acttccacct ccttcctcaa gattgccaat ctattgggtt ctccaaggtt 60
<210> 5
   <211> 31
   <212> DNA
   <213> Artificial Sequence
<220>
   <223> synthetic oligonucleotide
<400> 5
   gcatggccaa tctattgggt tctccaaggt t 31
<210> 6
   <211> 62
   <212> DNA
   <213> Artificial Sequence
<220>
   <223> synthetic oligonucleotide
<400> 6
   gccaccacct acttccacct cctccctcat tgcatggcca atctattggg ttctccaagg 60
   tt 62
<210> 7
   <211> 76
   <212> DNA
   <213> Glycine max
<400> 7
   atttctcatg gaaaataagc catcgccgcc atcactccaa cacaggttcc cttgaccgtg 60
   atgaagtgtt tgtccc 76
<210> 8
   <211> 68
   <212> DNA
   <213> Artificial Sequence
<220>
   <223> synthetic oligonucleotide
<400> 8
   atttctcatg gaaaataagc catcgccgcc atcactccaa cacttgaccg tgatgaagtg 60
   tttgtccc 68
<210> 9
   <211> 46
   <212> DNA
   <213> Artificial Sequence
<220>
   <223> synthetic oligonucleotide
<400> 9
   atttctcatg gaaaataagc catcgccgcg atgaagtgtt tgtccc 46
<210> 10
   <211> 70
   <212> DNA
   <213> Artificial Sequence
<220>
   <223> synthetic oligonucleotide
<400> 10
   atttctcatg gaaaataagc catcgccgcc accactccaa cacccttgac cgtgatgaag 60
   tgtttgtccc 70
<210> 11
   <211> 15
   <212> DNA
   <213> Artificial Sequence
<220>
   <223> synthetic oligonucleotide
<400> 11
   tgaagtgttt gtccc 15
<210> 12
   <211> 54
   <212> DNA
   <213> Artificial Sequence
<220>
   <223> synthetic oligonucleotide
<400> 12
   atttctcatg gaaaataaca cgggttccct tgaccgtgat gaagtgtttg tccc 54
<210> 13
   <211> 77
   <212> DNA
   <213> Glycine max
<400> 13
   atttctcatg gaaaataagc catcgccgcc atcactccaa cacaggttcc cttgaccgtg 60
   atgaagtgtt tgtccca                                                77
<210> 14
   <211> 27
   <212> DNA
   <213> Artificial Sequence
<220>
   <223> synthetic oligonucleotide
<400> 14
   cttgaccgtg atgaagtgtt tgtccca 27
<210> 15
   <211> 77
   <212> DNA
   <213> Glycine max
<400> 15
   atttctcatg gaaaataagc catcgccgcc accactccaa cacgggttcc cttgaccgtg 60
   atgaagtgtt tgtccca                                                77
<210> 16
   <211> 54
   <212> DNA
   <213> Artificial Sequence
<220>
   <223> synthetic oligonucleotide
<400> 16
   atttctcatg gaaaataagc catcgccctt gaccgtgatg aagtgtttgt ccca 54
<210> 17
   <211> 63
   <212> DNA
   <213> Artificial Sequence
<220>
   <223> synthetic oligonucleotide
<400> 17
   atttctcatg gaaaataagc catcgcctca ggttcccttg accgtgatga agtgtttgtc 60
   cca                                                               63
<210> 18
   <211> 23
   <212> DNA
   <213> Glycine max
<400> 18
   gccaccacct acttccacct cct 23
<210> 19
   <211> 16
   <212> DNA
   <213> Glycine max
<400> 19
   attgcatggc caatct 16
<210> 20
   <211> 23
   <212> DNA
   <213> Glycine max
<400> 20
   gccaccacct acttccacct cct 23
<210> 21
   <211> 16
   <212> DNA
   <213> Glycine max
<400> 21
   attgcatggc caatct 16
<210> 22
   <211> 25
   <212> DNA
   <213> Glycine max
<400> 22
   acattgccac cacctacttc cacct 25
<210> 23
   <211> 16
   <212> DNA
   <213> Glycine max
<400> 23
   attgcatggc caatct 16
<210> 24
   <211> 25
   <212> DNA
   <213> Glycine max
<400> 24
   acattgccac cacctacttc cacct 25
<210> 25
   <211> 16
   <212> DNA
   <213> Glycine max
<400> 25
   attgcatggc caatct 16
<210> 26
   <211> 19
   <212> DNA
   <213> Glycine max
<400> 26
   ctcatggaaa ataagccat 19
<210> 27
   <211> 22
   <212> DNA
   <213> Glycine max
<400> 27
   accgtgatga agtgtttgtc cc 22
<210> 28
   <211> 19
   <212> DNA
   <213> Glycine max
<400> 28
   ctcatggaaa ataagccat 19
<210> 29
   <211> 22
   <212> DNA
   <213> Glycine max
<400> 29
   accgtgatga agtgtttgtc cc 22
<210> 30
   <211> 23
   <212> DNA
   <213> Glycine max
<400> 30
   atttctcatg gaaaataagc cat 23
<210> 31
   <211> 22
   <212> DNA
   <213> Glycine max
<400> 31
   accgtgatga agtgtttgtc cc 22
<210> 32
   <211> 23
   <212> DNA
   <213> Glycine max
<400> 32
   atttctcatg gaaaataagc cat 23
<210> 33
   <211> 22
   <212> DNA
   <213> Glycine max
<400> 33
   accgtgatga agtgtttgtc cc 22
<210> 34
   <211> 58
   <212> DNA
   <213> Glycine max
<400> 34
   gccaccacct acttccacct ccttcctcaa cccttttccc tcattgcatg gccaatct 58
<210> 35
   <211> 63
   <212> DNA
   <213> Glycine max
<400> 35
   acattgccac cacctacttc cacctccttc ctcaaccctt ttccctcatt gcatggccaa 60
   tct                                                               63
<210> 36
   <211> 72
   <212> DNA
   <213> Glycine max
<400> 36
   ctcatggaaa ataagccatc gccgccatca ctccaacaca ggttcccttg accgtgatga 60
   agtgtttgtc cc                                                     72
<210> 37
   <211> 9
   <212> PRT
   <213> Artificial Sequence
<220>
   <223> consensus
<400> 37
   Leu Ala Gly Leu Ile Asp Ala Asp Gly
    1               5
<210> 38
   <211> 1331
   <212> DNA
   <213> Glycine max
<400> 38
   atggtcatga tttcactctc tctaatctct ccattcattt tgtagttgtc attatcttta 60
   gatttttcac tacctggttt aaaattgagg gattgtagtt ctgttggtac atattacaca 120
   ttcagcaaaa caactgaaac tcaactgaac ttgtttatac tttgacacag ggtctagcaa 180
   aggaaacaac aatgggaggt agaggtcgtg tggccaaagt ggaagttcaa gggaagaagc 240
   ctctctcaag ggttccaaac acaaagccac cattcactgt tggccaactc aagaaagcaa 300
   ttccaccaca ctgctttcag cgctccctcc tcacttcatt ctcctatgtt gtttatgacc 360
   tttcatttgc cttcattttc tacattgcca ccacctactt ccacctcctt cctcaaccct 420
   tttccctcat tgcatggcca atctattggg ttctccaagg ttgccttctc actggtgtgt 480
   gggtgattgc tcacgagtgt ggtcaccatg ccttcagcaa gtaccaatgg gttgatgatg 540
   ttgtgggttt gacccttcac tcaacacttt tagtccctta tttctcatgg aaaataagcc 600
   atcgccgcca tcactccaac acaggttccc ttgaccgtga tgaagtgttt gtcccaaaac 660
   caaaatccaa agttgcatgg ttttccaagt acttaaacaa ccctctagga agggctgttt 720
   ctcttctcgt cacactcaca atagggtggc ctatgtattt agccttcaat gtctctggta 780
   gaccctatga tagttttgca agccactacc acccttatgc tcccatatat tctaaccgtg 840
   agaggcttct gatctatgtc tctgatgttg ctttgttttc tgtgacttac tctctctacc 900
   gtgttgcaac cctgaaaggg ttggtttggc tgctatgtgt ttatggggtg cctttgctca 960
   ttgtgaacgg ttttcttgtg actatcacat atttgcagca cacacacttt gccttgcctc 1020
   attacgattc atcagaatgg gactggctga agggagcttt ggcaactatg gacagagatt 1080
   atgggattct gaacaaggtg tttcatcaca taactgatac tcatgtggct caccatctct 1140
   tctctacaat gccacattac catgcaatgg aggcaaccaa tgcaatcaag ccaatattgg 1200
   gtgagtacta ccaatttgat gacacaccat tttacaaggc actgtggaga gaagcgagag 1260
   agtgcctcta tgtggagcca gatgaaggaa catccgagaa gggcgtgtat tggtacagga 1320
   acaagtattg a                                                      1331
<210> 39
   <211> 1324
   <212> DNA
   <213> Glycine max
<400> 39
   atggtcatga tttcactctc tctaatctgt cacttccctc cattcatttt gtacttctca 60
   tatttttcac ttcctggttg aaaattgtag ttctcttggt acatactagt attagacatt 120
   cagcaacaac aactgaactg aacttcttta tactttgaca cagggtctag caaaggaaac 180
   aataatggga ggtggaggcc gtgtggccaa agttgaaatt cagcagaaga agcctctctc 240
   aagggttcca aacacaaagc caccattcac tgttggccaa ctcaagaaag ccattccacc 300
   gcactgcttt cagcgttccc tcctcacttc attgtcctat gttgtttatg acctttcatt 360
   ggctttcatt ttctacattg ccaccaccta cttccacctc ctccctcacc ccttttccct 420
   cattgcatgg ccaatctatt gggttctcca aggttgcatt cttactggcg tgtgggtgat 480
   tgctcacgag tgtggtcacc atgccttcag caagtaccca tgggttgatg atgttatggg 540
   tttgaccgtt cactcagcac ttttagtccc ttatttctca tggaaaataa gccatcgccg 600
   ccaccactcc aacacgggtt cccttgaccg tgatgaagtg tttgtcccaa aaccaaaatc 660
   caaagttgca tggtacacca agtacctgaa caaccctcta ggaagggctg cttctcttct 720
   catcacactc acaatagggt ggcctttgta tttagccttc aatgtctctg gcagacccta 780
   tgatggtttt gctagccact accaccctta tgctcccata tattcaaatc gtgagaggct 840
   tttgatctat gtctctgatg ttgctttgtt ttctgtgact tacttgctct accgtgttgc 900
   aactatgaaa gggttggttt ggctgctatg tgtttatggg gtgccattgc tcattgtgaa 960
   cggttttctt gtgaccatca catatctgca gcacacacac tatgccttgc ctcactatga 1020
   ttcatcagaa tgggattggc tgaggggtgc tttggcaact atggacagag attatggaat 1080
   tctgaacaag gtgtttcacc acataactga tactcatgtg gctcaccatc ttttctctac 1140
   aatgccacat taccatgcaa cggaggcaac caatgcaatg aagccaatat tgggtgagta 1200
   ctaccgattt gatgacacac cattttacaa ggcactgtgg agagaagcaa gagagtgcct 1260
   ctatgtggag ccagatgaag gaacatccga gaagggcgtg tattggtaca ggaacaagta 1320
   ttga                                                              1324

## Description

### CROSS-REFERENCE TO RELATED APPLICATIONS

This application claims benefit of priority from U.S. Provisional Application Serial No. 61/885,213, filed on October 1, 2013, and U.S. Provisional Application Serial No. 61/790,655, filed on March 15, 2013.

### TECHNICAL FIELD

This document relates to materials and methods for making soybean plants that can be used to produce oil having a modified composition as compared to wild type plants.

### BACKGROUND

Soybean *(Glycine max)* is an important legume crop worldwide due to its ability to fix atmospheric nitrogen. Soybean also serves as a major source of animal feed protein, and its oil has uses ranging from cooking/frying to industrial uses and biodiesel. Typically, a hydrogenation process is used to increase heat stability and improve the shelf life and taste of soybean oil. However, hydrogenation increases the cost of production and also results in the formation of trans fats, which have been linked to cardiovascular disease in humans.

Pharn et al describe in International Journal of Plant Breeding Research, vol. 123, no. 5, 17 June 2011, on pages 793-802, a novel allele in a soybean plant introduction that offers an alternate means to produce soybean seed oil with 85% oleic acid content.

Pham et al describe in BMC Plant Biology, vol. 10, no. 1, 9 September 2010, on page 195 that mutant alleles of FAD2-1A arid FAD2-1B combine to produce soybeans with the high oleic acid seed oil trait.

Cermak et al. describe in Nucleic Acids Research, vol. 39, no. 12, 1 July 2011, on pages 1-11 an efficient design and assemble of custom transcription activator-like (TAL) effector endonuclease-based constructs for DNA targeting.

Mahfouz et al describe in GM Crops, vol. 2, no. 2, 1 April 2011, on pages 99-103 uses of TAL effector endonucleases in genetically modified crops.

Curtin et al describe in Plant Physiology, vol. 156, no. 2, 4 April 2011, on pages 466-473 targeted mutagenesis of duplicated genes in soybean with zinc-finger nucleases.

### SUMMARY

The present invention is defined by the independent claims. The dependent claims depict additional embodiments of the invention.

Described herein are materials and methods for modifying soybean oil composition by reducing or eliminating expression of the delta-twelve fatty acid desaturase 2 (FAD2) 1A and 1B genes without the use of transgenesis.

The methods described herein utilize sequence-specific, rare-cutting (TAL) effector endonucleases to introduce mutations in the FAD2-1A and/or FAD2-1B coding sequences, thereby knocking out gene function. These methods can mediate FAD2-1A/1B silencing without insertion of a transgene. Plants containing transgenes are highly regulated in certain jurisdictions, such as Europe, and in other jurisdictions, costs to obtain regulatory approval are very high. The methods described herein also can expedite the production of new varieties, and can be more cost-effective than transgenic or traditional breeding approaches.

In one aspect, this document features a method for producing soybean oil having increased oleic acid content and reduced linoleic acid content. The method includes (a) providing a soybean plant or plant part including (i) a deletion in one or more FAD2-1A alleles, wherein said deletion in the one or more FAD2-1A alleles was induced by transcription activator-like (TAL) effector endonucleases binding to SEQ ID NOs: 32 and 33, and (ii) a deletion in one or more FAD2-1B alleles, wherein said deletion in the one or more FAD2-1B alleles was induced by TAL effector endonucleases binding to SEQ ID NOs: 32 and 33; and (b) producing oil from the plant or plant part. In some embodiments, the plant or plant part does not contain a transgene. In some embodiments, the one or more FAD2-1B alleles comprise SEQ ID NO: 16.

In another aspect, this document features a method for making a soybean plant having a mutation in each FAD2-1A allele and a mutation in each FAD2-1B allele. The method includes (a) contacting a population of soybean plant cells comprising functional FAD2-1A and FAD2-1B alleles with TAL effector endonucleases targeted to SEQ If) NOs: 32 and 33, (b) selecting, from said population, a cell in which each FAD2-1A allele and each FAD2-1B allele comprises a deletion, wherein said deletion in each FAD2-1A allele was induced by said TAL effector endonucleases targeted to SEQ ID NOs: 32 and 33, and wherein said deletion in each FAD2-1B allele was induced by said TAL effector endonucleases targeted to SEQ ID NOs: 32 and 33, and (c) regenerating the selected plant cell into a soybean plant. The soybean plant cells can contain cotyledon cells. The method can include transforming cotyledon cells with one or more vectors encoding the TAL effector endonucleases. The method can further include culturing the plant cells to generate plant lines. In some embodiments, each FAD2-1 B allele in said selected cell comprises SEQ ID NO: 16.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict between the present specification and a reference mentioned herein, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF DRAWINGS

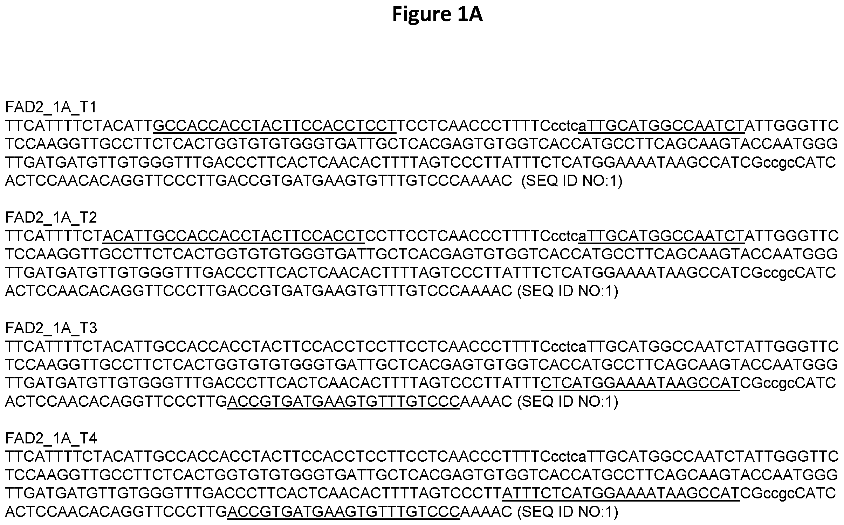

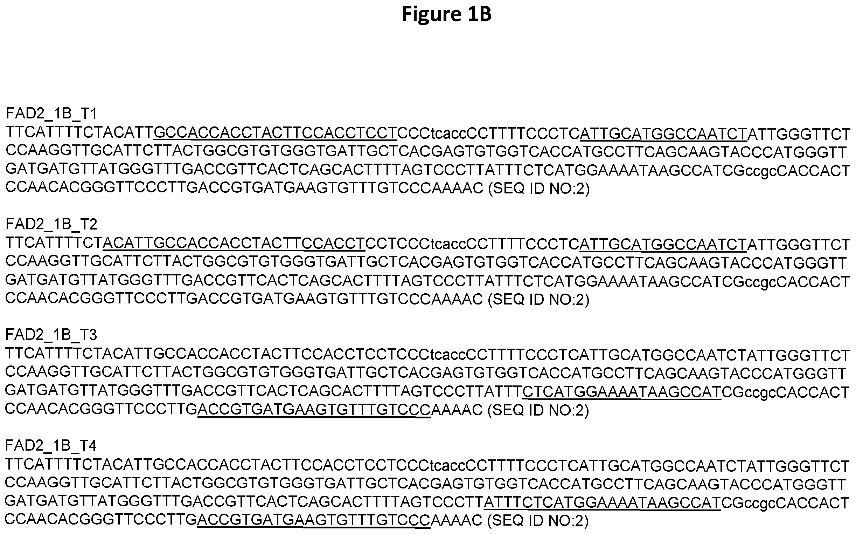

FIGS. 1A and 1B show DNA sequences for various TAL effector endonuclease target sites in FAD2-1A and FAD2-1B. DNA sequence from the FAD2-1A gene (SEQ ID NO:1) is shown in FIG. 1A, and DNA sequence from the FAD2-1B gene (SEQ ID NO:2) is shown in FIG. 1B. The underlined sequences indicate target sites for TAL effector endonucleases, designated T1 to T4. Lower case letters denote restriction endonuclease sites used to screen for TAL effector endonuclease-induced mutations.

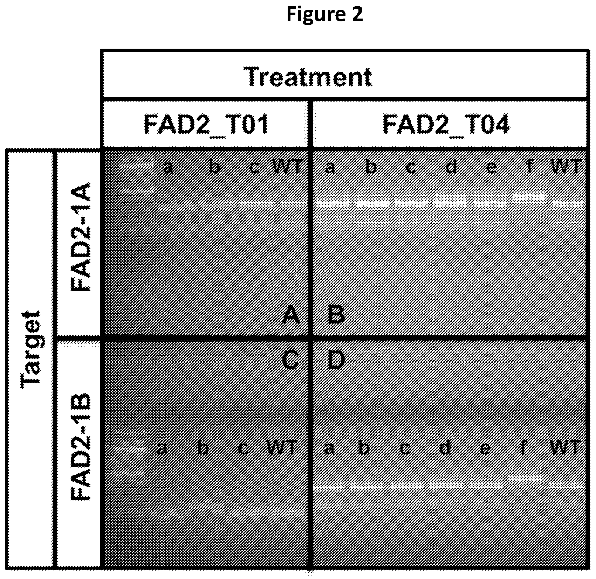

FIG. 2 is a picture of a gel showing products from a PCR enrichment assay used to screen soybean hairy roots for TAL effector endonuclease-induced mutations in FAD2-1A and FAD2-1B.

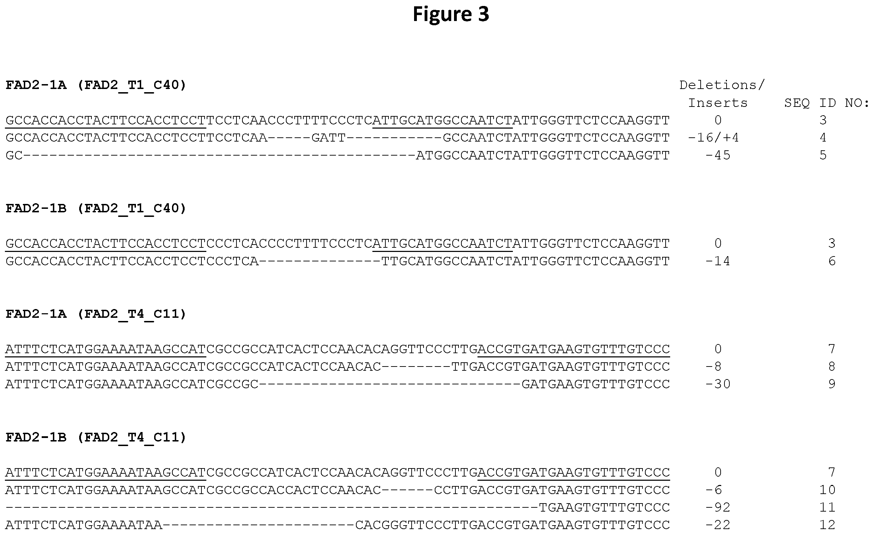

FIG. 3 shows exemplary DNA sequences of TAL effector endonuclease-induced mutations in the FAD2-1A and FAD2-1B genes in soybean hairy roots. The top line of each panel shows the DNA sequence of the recognition site for the TAL effector endonucleases (underlined) in FAD2-1A (top two panels) and FAD2-1B (bottom two panels). The other sequences show representative mutations that were induced by imprecise non-homologous end-joining (NHEJ), with the sizes of deletions given on the right.

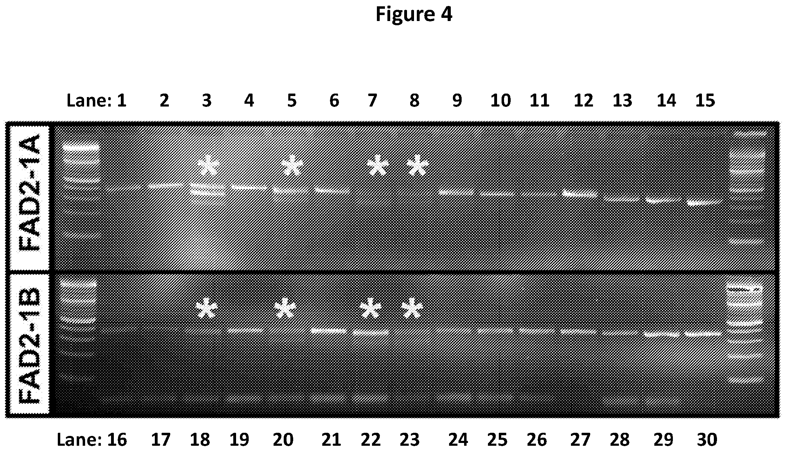

FIG. 4 is a picture of a gel showing the result of a T7E1 assay performed on regenerated soybean plants to identify TAL effector endonuclease-induced mutations in FAD2-1A and FAD2-1B. Asterisks indicate samples with evident mutations. Table 4A herein provides information about which regenerated plant and which particular FAD2-1 gene were analyzed in each lane of the gel. The first and last lanes contain molecular length markers and are not numbered. DNA samples listed as "uncut" in Table 4A were not treated with T7E1.

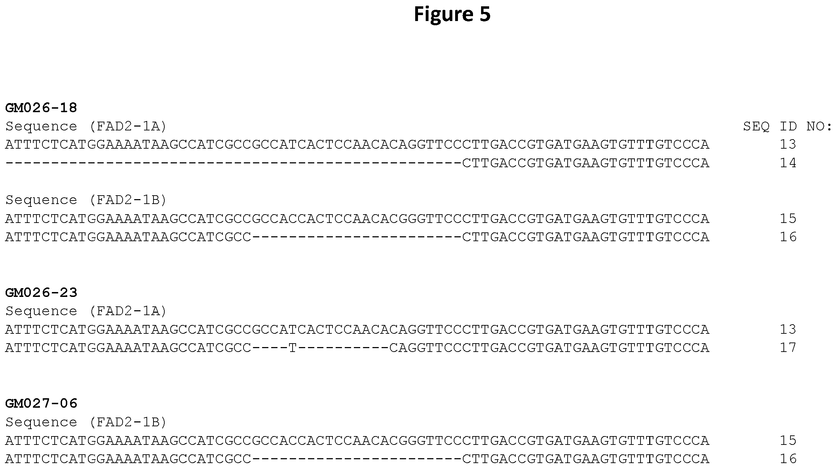

FIG. 5 shows DNA sequences of TAL effector endonuclease-induced mutations in FAD2-1A and FAD2-1B. These mutations were genetically transmissible.

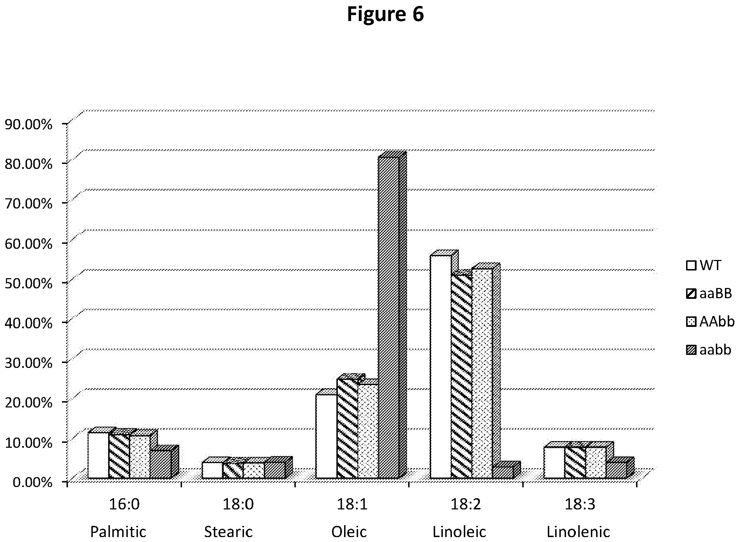

FIG. 6 shows the results of fatty acid composition analyses for different progeny derived from GM02 6-18. Plants with the aaBB genotype have mutations in both alleles of FAD2-1A. Plants with the AAbb genotype have mutations in both alleles of FAD2-1B. The aabb plants have mutations in both alleles of FAD2-1A and FAD2-1B.

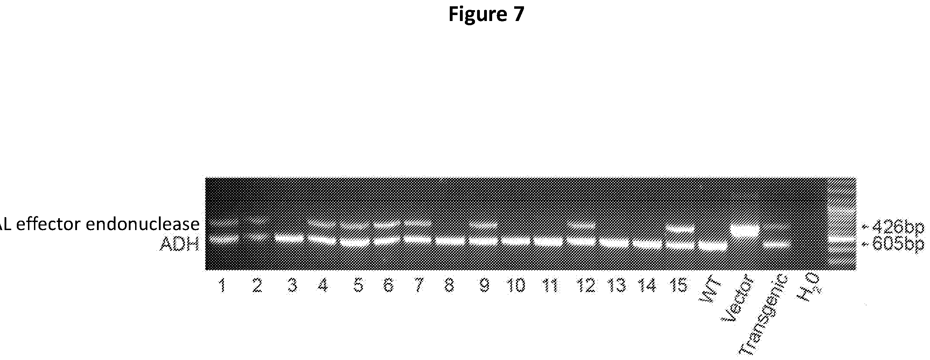

FIG. 7 shows segregation of the TAL effector endonuclease transgene in FAD2-1 mutants. The gel image shows PCR products obtained with primer sets for the TAL effector endonuclease coding sequences and the soybean alcohol dehydrogenase (ADH) gene, respectively. Labels below the gel image represent: 1-15, individual T2 mutant lines; WT, DNA from a non-transgenic wild-type soybean plant; vector, vector DNA used in transformation; transgenic, positive control DNA from a transgenic plant containing the T-DNA vector; H₂O, negative control without any DNA.

### DETAILED DESCRIPTION

Commodity soybean oil is made up of five fatty acids: palmitic acid (10%), stearic acid (4%), oleic acid (18%), linoleic acid (55%) and linolenic acid (13%). Plant oils with high oleic acid content may require less processing to improve stability and/or taste. Such oils also may be healthier, as well as better suited for the production of biodiesel. Soybean oil having an oleic acid content of greater than 55% and a linoleic acid content of less than 10% can be particularly useful. Traditional breeding and mutagenesis strategies have been used to generate soybean varieties containing elevated levels of oleic acid, but these varieties have reduced yield and thus have not been acceptable to farmers.

The enzymes responsible for the biosynthetic progression from palmitic acid to linolenic acid have been identified. For example, the FAD2 genes are responsible for converting oleic acid precursors to linoleic acid precursors during oil accumulation in developing soybean seeds. Due to the ancient polyploidization of soybean, two copies of FAD2 (FAD2-1A and FAD2-1B) exist in the soybean genome. These genes have about 95% sequence identity at the DNA level, and the encoded proteins have about 99% sequence identity at the amino acid level. Plants homozygous for naturally occurring FAD2-1B mutant alleles can have a modest increase (20.5% to 29.4%) in oleic acid composition, as described elsewhere (Pham et al., BMC Plant Biol. 10:195, 2010). Mutations in FAD2-1A have been developed through X-ray mutagenesis and TILLing, to produce seeds containing up to 50% oleic acid (Sandhu et al., JAOCS 84:229-235, 2007), and mutating both the FAD2-1A and FAD2-1B alleles resulted in oil with an oleic acid content of 82.2% (Pham et al., *supra*)*.*

This document provides soybean plants that have reduced (e.g., lack) FAD2-1A and/or FAD2-1B activity, as well methods for generating such plants, and oil derived from such plants. The methods described herein can be used to generate soybean varieties having oil with an increased oleic acid component of at least 30% (e.g., at least 35%, at least 40%, at least 45%, at least 50%, at least 55%, at least 60%, at least 65%, at least 70%, at least 75%, or at least 80%), and a reduction in linoleic acid component to 10% or less (e.g., 8% or less, 5% or less, 4% or less, 3% or less, 2% or less, 1% or less, or 0.5% or less). Removing the gene products of FAD2-1A/1B can severely reduce the conversion of oleic acid precursors to linoleic acid precursors in soybean seeds.

To accomplish the complete elimination of FAD2-1A/1B expression, for example, an engineered, rare-cutting nuclease was designed to recognize a conserved region of both FAD2-1 genes and create a double-strand break. Improper repair due to Non-Homologous End Joining (NHEJ) at the DNA break site generates missense and/or nonsense mutations in the FAD2-1A/1B coding regions, rendering the FAD2-1A/1B RNA transcripts unstable and targeted for degradation prior to translation.

Representative examples of naturally occurring soybean FAD2-1A and FAD2-1B nucleotide sequences are shown in Table 5. The soybean plants, cells, plant parts, seeds, and progeny thereof provided herein can have a mutation in each of the endogenous FAD2-1A and FAD2-1B alleles, such that expression of each gene is reduced or completely inhibited. Alternatively, the soybean plants, cells, plant parts, seeds, and progeny thereof provided herein may have a mutation in at least one FAD2-1A allele and/or in at least one FAD2-1B allele, such that expression of each gene is reduced or completely inhibited. The soybean plants, cells, parts, seeds, and progeny can have increased levels of oleic acid and reduced levels of linoleic acid as compared to wild type soybean plants, cells, parts, seeds, and progeny.

The plants, plant cells, plant parts, seeds, and plant progeny provided herein are generated using a TAL effector endonuclease system to make targeted knockouts in the FAD2-1A and/or FAD2-1B genes. Thus, this disclosure provides materials and methods for using TAL effector endonucleases to generate plants and related products (e.g., seeds and plant parts) that are particularly suitable for production of soybean oil with increased oleic acid content and decreased linoleic acid content.

"Plants" and "plant parts" refers to cells, tissues, organs, seeds, and severed parts (e.g., roots, leaves, and flowers) that retain the distinguishing characteristics of the parent plant. "Seed" refers to any plant structure that is formed by continued differentiation of the ovule of the plant, following its normal maturation point at flower opening, irrespective of whether it is formed in the presence or absence of fertilization and irrespective of whether or not the seed structure is fertile or infertile.

The term "allele(s)" means any of one or more alternative forms of a gene at a particular locus. In a diploid (or amphidiploid) cell of an organism, alleles of a given gene are located at a specific location or locus on a chromosome. One allele is present on each chromosome of the pair of homologous chromosomes. "Heterozygous" alleles are two different alleles residing at a specific locus, positioned individually on corresponding pairs of homologous chromosomes. "Homozygous" alleles are two identical alleles residing at a specific locus, positioned individually on corresponding pairs of homologous chromosomes in the cell.

"Wild type" as used herein refers to a typical form of a plant or a gene as it most commonly occurs in nature. A "wild type FAD2-1A allele" is a naturally occurring FAD2-1A allele (e.g., as found within naturally occurring soybean plants) that encodes a functional FAD2-1A protein, while a "mutant FAD2-1A allele" is a FAD2-1A allele that does not encode a functional FAD2-1A protein. Such a "mutant FAD2-1A allele" can include one or more mutations in its nucleic acid sequence, where the mutation(s) result in no detectable amount of functional FAD2-1A protein in the plant or plant cell *in vivo.*

The term "rare-cutting endonucleases" herein refers to natural or engineered proteins having endonuclease activity directed to nucleic acid sequences having a recognition sequence (target sequence) about 12-40 bp in length (e.g., 14-40 bp in length). Typical rare-cutting endonucleases cause cleavage inside their recognition site, leaving 4 nt staggered cut with 3'OH or 5'OH overhangs. These rare-cutting endonucleases may be meganucleases, such as wild type or variant proteins of homing endonucleases, more particularly belonging to the dodecapeptide family (LAGLIDADG (SEQ ID NO:37); *see,* WO 2004/067736) or may result from fusion proteins that associate a DNA binding domain and a catalytic domain with cleavage activity. TAL-effector endonucleases and zinc-finger-nucleases (ZFN) are examples of fusions of DNA binding domains with the catalytic domain of the endonuclease *Fok***I***.* Customized TAL effector endonucleases are commercially available under the trade name TALEN™ (Cellectis, Paris, France). For a review of rare-cutting endonucleases, see Baker, Nature Methods 9:23-26, 2012.

"Mutagenesis" as used herein refers to processes in which mutations are introduced into a selected DNA sequence. Mutations induced by endonucleases generally are obtained by a double strand break, which results in insertions or deletions ("indels") that can be detected by deep-sequencing analysis. Such mutations typically are deletions of several base pairs, and have the effect of inactivating the mutated allele. In the methods described herein, for example, mutagenesis occurs via double stranded DNA breaks made by TAL effector endonucleases targeted to selected DNA sequences in a plant cell. Such mutagenesis results in "TAL effector endonuclease-induced mutations" (e.g., TAL effector endonuclease-induced knockouts) and reduced expression of the targeted gene. Following mutagenesis, plants can be regenerated from the treated cells using known techniques (e.g., planting seeds in accordance with conventional growing procedures, followed by self-pollination).

In some cases, a nucleic acid can have a nucleotide sequence with at least about 75 percent sequence identity to a representative FAD2-1A or FAD2-1B nucleotide sequence. For example, a nucleotide sequence can have at least 75, at least 80, at least 85, at least 90, at least 91, at least 92, at least 93, at least 94, at least 95, at least 96, at least 97, at least 98, or at least 99 percent sequence identity to a representative, naturally occurring FAD2-1A or FAD2-1B nucleotide sequence as set forth in Table 5.

The percent sequence identity between a particular nucleic acid or amino acid sequence and a sequence referenced by a particular sequence identification number is determined as follows. First, a nucleic acid or amino acid sequence is compared to the sequence set forth in a particular sequence identification number using the BLAST 2 Sequences (Bl2seq) program from the stand-alone version of BLASTZ containing BLASTN version 2.0.14 and BLASTP version 2.0.14. This stand-alone version of BLASTZ can be obtained online at fr.com/blast or at ncbi.nlm.nih.gov. Instructions explaining how to use the Bl2seq program can be found in the readme file accompanying BLASTZ. Bl2seq performs a comparison between two sequences using either the BLASTN or BLASTP algorithm. BLASTN is used to compare nucleic acid sequences, while BLASTP is used to compare amino acid sequences. To compare two nucleic acid sequences, the options are set as follows: -i is set to a file containing the first nucleic acid sequence to be compared (e.g., C:\seq1.txt); -j is set to a file containing the second nucleic acid sequence to be compared (e.g., C:\seq2.txt); - p is set to blastn; -o is set to any desired file name (e.g., C:\output.txt); -q is set to -1; - r is set to 2; and all other options are left at their default setting. For example, the following command can be used to generate an output file containing a comparison between two sequences: C:\Bl2seq -i c:\seq1.txt -j c:\seq2.txt -p blastn -o c:\output.txt -q -1 -r 2. To compare two amino acid sequences, the options of Bl2seq are set as follows: -i is set to a file containing the first amino acid sequence to be compared (e.g., C:\seq1.txt); -j is set to a file containing the second amino acid sequence to be compared (e.g., C:\seq2.txt); -p is set to blastp; -o is set to any desired file name (e.g., C:\output.txt); and all other options are left at their default setting. For example, the following command can be used to generate an output file containing a comparison between two amino acid sequences: C:\Bl2seq -i c:\seq1.txt -j c:\seq2.txt -p blastp -o c:\output.txt. If the two compared sequences share homology, then the designated output file will present those regions of homology as aligned sequences. If the two compared sequences do not share homology, then the designated output file will not present aligned sequences.

Once aligned, the number of matches is determined by counting the number of positions where an identical nucleotide or amino acid residue is presented in both sequences. The percent sequence identity is determined by dividing the number of matches either by the length of the sequence set forth in the identified sequence (e.g., SEQ ID NO: 1), or by an articulated length (e.g., 100 consecutive nucleotides or amino acid residues from a sequence set forth in an identified sequence), followed by multiplying the resulting value by 100. For example, a nucleic acid sequence that has 250 matches when aligned with the sequence set forth in SEQ ID NO: 1 is 86.5 percent identical to the sequence set forth in SEQ ID NO: 1 (i.e., 250 ÷ 289 x 100 = 86.5). It is noted that the percent sequence identity value is rounded to the nearest tenth. For example, 75.11, 75.12, 75.13, and 75.14 is rounded down to 75.1, while 75.15, 75.16, 75.17, 75.18, and 75.19 is rounded up to 75.2. It also is noted that the length value will always be an integer.

Methods for selecting endogenous target sequences and generating TAL effector endonucleases targeted to such sequences can be performed as described elsewhere. *See,* for example, PCT Publication No. WO 2011/072246. TAL effectors are found in plant pathogenic bacteria in the genus *Xanthomonas.* These proteins play important roles in disease, or trigger defense, by binding host DNA and activating effector-specific host genes *(see,* e.g., Gu et al., Nature 435:1122-1125, 2005; Yang et al., Proc. Natl. Acad. Sci. USA 103:10503-10508, 2006; Kay et al. Science 318:648-651, 2007; Sugio et al., Proc. Natl. Acad. Sci. USA 104:10720-10725, 2007; and Römer et al. Science 318:645-648, 2007). Specificity depends on an effector-variable number of imperfect, typically 34 amino acid repeats (Schornack et al., J. Plant Physiol. 163:256-272, 2006; and WO 2011/072246). Polymorphisms are present primarily at repeat positions 12 and 13, which are referred to herein as the repeat variable-diresidue (RVD).

The RVDs of TAL effectors correspond to the nucleotides in their target sites in a direct, linear fashion, one RVD to one nucleotide, with some degeneracy and no apparent context dependence. This mechanism for protein-DNA recognition enables target site prediction for new target specific TAL effectors, as well as target site selection and engineering of new TAL effectors with binding specificity for the selected sites.

TAL effector DNA binding domains can be fused to other sequences, such as endonuclease sequences, resulting in chimeric endonucleases targeted to specific, selected DNA sequences, and leading to subsequent cutting of the DNA at or near the targeted sequences. Such cuts (i.e., double-stranded breaks) in DNA can induce mutations into the wild type DNA sequence via NHEJ or homologous recombination, for example. In some cases, TAL effector endonucleases can be used to facilitate site directed mutagenesis in complex genomes, knocking out or otherwise altering gene function with great precision and high efficiency. As described in the Examples below, TAL effector endonucleases targeted to the soybean FAD2-1A and FAD2-1B alleles can be used to mutagenize the endogenous genes, resulting in plants with reduced expression (e.g., without detectable expression) of these genes. The fact that some endonucleases (e.g., *Fok***I***)* function as dimers can be used to enhance the target specificity of the TAL effector endonuclease. For example, in some cases a pair of TAL effector endonuclease monomers targeted to different DNA sequences (e.g., the underlined target sequences shown in FIGS. 1A and 1B) can be used. When the two TAL effector endonuclease recognition sites are in close proximity, as depicted in FIGS. 1A and 1B, the inactive monomers can come together to create a functional enzyme that cleaves the DNA. By requiring DNA binding to activate the nuclease, a highly site-specific restriction enzyme can be created.

The term "expression" as used herein refers to the transcription of a particular nucleic acid sequence to produce sense or antisense mRNA, and/or the translation of a sense mRNA molecule to produce a polypeptide (e.g., a therapeutic protein), with or without subsequent post-translational events.

"Reducing the expression" of a gene or polypeptide in a plant or a plant cell includes inhibiting, interrupting, knocking-out, or knocking-down the gene or polypeptide, such that transcription of the gene and/or translation of the encoded polypeptide are reduced as compared to a corresponding wild type plant or plant cell. Expression levels can be assessed using methods such as, for example, reverse transcription-polymerase chain reaction (RT-PCR), Northern blotting, dot-blot hybridization, *in situ* hybridization, nuclear run-on and/or nuclear run-off, RNase protection, or immunological and enzymatic methods such as ELISA, radioimmunoassay, and western blotting.

Methods for using TAL effector endonucleases to generate plants, plant cells, or plant parts having mutations in endogenous genes include, for example, those described in the Examples herein. For example, nucleic acids encoding TAL effector endonucleases targeted to selected FAD2-1A or FAD2-1B sequences (e.g., the FAD2-1A sequences shown in FIG. 1A or the FAD2-1B sequences shown in FIG. 1B) can be transformed into plant cells (e.g., cells in cotyledons), where they can be expressed. The cells subsequently can be analyzed to determine whether mutations have been introduced at the target site(s), through nucleic acid-based assays or protein-based assays to detect expression levels as described above, for example, or using nucleic acid-based assays (e.g., PCR and DNA sequencing, or PCR followed by a T7E1 assay; Mussolino et al., Nucl. Acids Res. 39:9283-9293, 2011) to detect mutations at the genomic loci.

The mutagenized population, or a subsequent generation of that population, can be screened for a desired trait(s) (e.g., plants that have altered oil composition) that results from the mutations. Alternatively, the mutagenized population, or a subsequent generation of that population, can be screened for a mutation in a FAD2-1A or FAD2-1B gene. For example, the progeny M₂ generation of M₁ plants may be evaluated for the presence of a mutation in a FAD2-1A or FAD2-1B gene. A "population" is any group of individuals that share a common gene pool. As used herein, "M₀" refers to plant cells (and plants grown therefrom) exposed to a TAL effector nuclease, while "M₁" refers to seeds produced by self-pollinated M₀ plants, and plants grown from such seeds. "M₂" is the progeny (seeds and plants) of self-pollinated M₁ plants, "M₃" is the progeny of self-pollinated M₂ plants, and "M₄" is the progeny of self-pollinated M₃ plants. "M₅" is the progeny of self-pollinated M₄ plants. "M₆", "M₇", etc. are each the progeny of self-pollinated plants of the previous generation. The term "selfed" as used herein means self-pollinated.

One or more M₁ soybean plants can be obtained from individual, mutagenized M₀ plant cells (and plants grown therefrom), and at least one of the plants can be identified as containing a mutation in a FAD2-1A or FAD2-1B gene. An M₁ soybean plant may be heterozygous for a mutant allele at a FAD2-1A and/or a FAD2-1B locus and, due to the presence of the wild-type allele, exhibit delta-twelve fatty acid desaturase activity. Alternatively, an M₁ soybean plant may have a mutant allele at a FAD2-1A or FAD2-1B locus and exhibit the phenotype of lacking delta-twelve fatty acid desaturase activity. Such plants may be heterozygous and lack delta-twelve fatty acid desaturase activity due to phenomena such a dominant negative suppression, despite the presence of the wild-type allele, or may be homozygous due to independently induced mutations in both alleles at the FAD2-1A or FAD2-1B locus.

A soybean plant carrying mutant FAD2-1A and FAD2-1B alleles can be used in a plant breeding program to create novel and useful lines and varieties. Thus, in some examples not forming part of the claimed invention, an M₁, M₂, M₃, or later generation soybean plant containing at least one mutation in a FAD2-1A and at least one mutation in a FAD2-1B gene is crossed with a second soybean plant, and progeny of the cross are identified in which the FAD2-1A and FAD2-1B gene mutations are present. It will be appreciated that the second soybean plant can contain the same FAD2-1A and FAD2-1B mutations as the plant to which it is crossed, different FAD2-1A and FAD2-1B mutations, or be wild-type at the FAD2-1A and/or FAD2-1B loci.

Breeding can be carried out via known procedures. DNA fingerprinting, SNP or similar technologies may be used in a marker-assisted selection (MAS) breeding program to transfer or breed mutant FAD2-1A and FAD2-1B alleles into other soybean plants. For example, a breeder can create segregating populations from hybridizations of a genotype containing a mutant allele with an agronomically desirable genotype. Plants in the F₂ or backcross generations can be screened using markers developed from FAD2-1A and FAD2-1B sequences or fragments thereof. Plants identified as possessing the mutant allele can be backcrossed or self-pollinated to create a second population to be screened. Depending on the expected inheritance pattern or the MAS technology used, it may be necessary to self-pollinate the selected plants before each cycle of backcrossing to aid identification of the desired individual plants. Backcrossing or other breeding procedure can be repeated until the desired phenotype of the recurrent parent is recovered.

Successful crosses yield F₁ plants that are fertile and that can be backcrossed with one of the parents if desired. In some examples not forming part of the claimed invention, a plant population in the F₂ generation is screened for FAD2-1A and FAD2-1B gene expression, e.g., a plant is identified that fails to express FAD2-1A and FAD2-1B due to the absence of a FAD2-1A and FAD2-1B genes according to standard methods, for example, using a PCR method with primers based upon the nucleotide sequence information for FAD2-1A and FAD2-1B described herein. Selected plants are then crossed with one of the parents and the first backcross (BC₁) generation plants are self-pollinated to produce a BC₁F₂ population that is again screened for variant FAD2-1A and FAD2-1B gene expression (e.g., null versions of the FAD2-1A and FAD2-1B genes). The process of backcrossing, self-pollination, and screening is repeated, for example, at least four times until the final screening produces a plant that is fertile and reasonably similar to the recurrent parent. This plant, if desired, can be self-pollinated, and the progeny subsequently can be screened again to confirm that the plant lacks FAD2-1A and FAD2-1B gene expression. Cytogenetic analyses of the selected plants optionally can be performed to confirm the chromosome complement and chromosome pairing relationships. Breeder's seed of the selected plant can be produced using standard methods including, for example, field testing, confirmation of the null condition for FAD2-1A and FAD2-1B, and/or analyses of oil to determine the level of oleic acid and linoleic acid.

In situations where the original F₁ hybrid resulting from the cross between a first, mutant soybean parent and a second, wild-type soybean parent, is hybridized or backcrossed to the mutant soybean parent, the progeny of the backcross can be self-pollinated to create a BC₁F₂ generation that is screened for the mutant FAD2-1A and FAD2-1B alleles.

The result of a plant breeding program using the mutant soybean plants described herein can be novel and useful lines and varieties. As used herein, the term "variety" refers to a population of plants that share constant characteristics which separate them from other plants of the same species. A variety is often, although not always, sold commercially. While possessing one or more distinctive traits, a variety can be further characterized by a very small overall variation between individuals within that variety. A "pure line" variety may be created by several generations of self-pollination and selection, or vegetative propagation from a single parent using tissue or cell culture techniques. A variety can be essentially derived from another line or variety. As defined by the International Convention for the Protection of New Varieties of Plants (December 2, 1961, as revised at Geneva on November 10, 1972, on October 23, 1978, and on March 19, 1991), a variety is "essentially derived" from an initial variety if: a) it is predominantly derived from the initial variety, or from a variety that is predominantly derived from the initial variety, while retaining the expression of the essential characteristics that result from the genotype or combination of genotypes of the initial variety; b) it is clearly distinguishable from the initial variety; and c) except for the differences which result from the act of derivation, it conforms to the initial variety in the expression of the essential characteristics that result from the genotype or combination of genotypes of the initial variety. Essentially derived varieties can be obtained, for example, by the selection of a natural or induced mutant, a somaclonal variant, a variant individual from plants of the initial variety, backcrossing, or transformation. A "line" as distinguished from a variety most often denotes a group of plants used non-commercially, for example in plant research. A line typically displays little overall variation between individuals for one or more traits of interest, although there may be some variation between individuals for other traits.

The methods provided herein can be used to produce plant parts (e.g., seeds) or plant products (e.g., oil) having increased oleic acid content and reduced linoleic acid content, as compared corresponding plant parts or products from wild type plants. The fatty acid content of a plant part or a plant product can be evaluated using standard methods, such as those described in Example 5 herein, for example.

The invention will be further described in the following examples, which do not limit the scope of the invention described in the claims.

### EXAMPLES

### Example 1 - Engineering sequence-specific nucleases to mutagenize the FAD2-1A and FAD2-1B genes

To completely inactivate or knock-out the alleles of FAD2-1A and FAD2-1B genes in *G*. *max,* sequence-specific nucleases were designed that target the protein coding region in the vicinity of the start codon. Eight TAL effector endonuclease pairs were designed to target the FAD2-1 gene family within the first 500bp of the coding sequence using software that specifically identifies TAL effector endonuclease recognition sites, such as TALE-NT 2.0 (Doyle et al., Nucl. Acids Res. 40:W117-122, 2012). The TAL effector endonuclease recognition sites for the FAD2-1 genes are underlined in FIG. 1 and are listed in Table 1. TAL effector endonucleases were synthesized using methods similar to those described elsewhere (Cermak et al., Nucl. Acids Res. 39:e82, 2011; Reyon et al., Nat. Biotechnol. 30:460-465, 2012; and Zhang et al., Nat. Biotechnol. 29:149-153, 2011).

### Example 2 - FAD2-1 TAL effector endonuclease activity in yeast

To assess the activity of the TAL effector endonucleases targeting the FAD2-1 genes, activity assays were performed in yeast by methods similar to those described elsewhere (Christian et al., Genetics 186:757-761, 2010). For these assays, a target plasmid was constructed with the TAL effector endonuclease recognition site cloned in a non-functional β-galactosidase reporter gene (Table 2). The target site was flanked by a direct repeat of β-galactosidase coding sequence such that if the reporter gene was cleaved by the TAL effector endonuclease, recombination would occur between the direct repeats and function would be restored to the β-galactosidase gene. β-galactosidase activity, therefore, served as a measure of TAL effector endonuclease cleavage activity. Results are summarized in Table 3. All of the FAD2-1 TAL effector endonuclease pairs displayed cleavage activity. Cleavage activities were normalized to the benchmark nuclease, I-SceI.

**Table 1**

| *TAL effector endonuclease target sequences* | | | | |
|---|---|---|---|---|
| **Gene** | **Target Sequence Left** | **SEQ ID NO:** | **Target Sequence Right** | **SEQ ID NO:** |
| FAD2_T01_C11 | GCCACCACCTACTTCCACCTCCT | 18 | ATTGCATGGCCAATCT | 19 |
| FAD2_T01_C40 | GCCACCACCTACTTCCACCTCCT | 20 | ATTGCATGGCCAATCT | 21 |
| FAD2_T02_C11 | ACATTGCCACCACCTACTTCCACCT | 22 | ATTGCATGGCCAATCT | 23 |
| FAD2_T02_C40 | ACATTGCCACCACCTACTTCCACCT | 24 | ATTGCATGGCCAATCT | 25 |
| FAD2_T03_C11 | CTCATGGAAAATAAGCCAT | 26 | ACCGTGATGAAGTGTTTGTCCC | 27 |
| FAD2_T03_C40 | CTCATGGAAAATAAGCCAT | 28 | ACCGTGATGAAGTGTTTGTCCC | 29 |
| FAD2_T04_C11 | ATTTCTCATGGAAAATAAGCCAT | 30 | ACCGTGATGAAGTGTTTGTCCC | 31 |
| FAD2_T04_C40 | ATTTCTCATGGAAAATAAGCCAT | 32 | ACCGTGATGAAGTGTTTGTCCC | 33 |

**Table 2**

| *FAD2 TAL effector endonuclease Target Sequences for Yeast Assay* | | |
|---|---|---|
| **TAL effector endonuclease target** | **TAL effector endonuclease target sequence** | **SEQ ID NO:** |
| FAD2 Target 1 | GCCACCACCTACTTCCACCTCCTTCCTCAACCCTTTTCCCTCATTGCATGGCCAATCT | 34 |
| FAD2 Target 2 | ACATTGCCACCACCTACTTCCACCTCCTTCCTCAACCCTTTTCCCTCATTGCATGGCCAATCT | 35 |
| FAD2 Target 3 | CTCATGGAAAATAAGCCATCGCCGCCATCACTCCAACACAGGTTCCCTTGACCGTGATGAAGTGTTTGTCCC | 36 |
| FAD2 Target 4 | ATTTCTCATGGAAAATAAGCCATCGccgcCATCACTCCAACACAGGTTCCCTTGACCGTGATGAAGTGTTTGTCCC | 7 |

**Table 3**

| *FAD2 TAL effector endonuclease activity in yeast* | | | | |
|---|---|---|---|---|
| | **Activity in yeast*** | | | |
| **TAL effector endonuclease subunit** | **FAD2_T1** | **FAD2_T2** | **FAD2_**T3 | **FAD2_**T4 |
| FAD2_T01_Left_C11 | 0.73 | 0.87 | 0.00 | 0.00 |
| FAD2_T01_Right_C11 | | | | |
| FAD2_T01_Left_C40 | 0.79 | 0.73 | 0.00 | 0.00 |
| FAD2_T01_Right_C40 | | | | |
| FAD2_T02_Left_C11 | 0.00 | 0.97 | 0.00 | 0.00 |
| FAD2_T01_Right_C11 | | | | |
| FAD2_T02_Left_C40 | 0.00 | 0.96 | 0.00 | 0.00 |
| FAD2_T01_Right_C40 | | | | |
| FAD2_T03_Left_C11 | 0.00 | 0.00 | 0.65 | 0.53 |
| FAD2_T03_Right_C11 | | | | |
| FAD2_T03_Left_C40 | 0.00 | 0.00 | 0.63 | 0.51 |
| FAD2_T03_Right_C40 | | | | |
| FAD2_T04_Left_C11 | 0.00 | 0.00 | 0.00 | 0.54 |
| FAD2_T03_Right_C11 | | | | |
| FAD2_T04_Left_C40 | 0.00 | 0.00 | 0.00 | 0.52 |
| FAD2_T03_Right_C40 | | | | |

| | | | | |
|---|---|---|---|---|
| *Normalized to I-SceI (max = 1.0) | | | | |

### Example 3 - TAL effector endonuclease expression and activity in soybean hairy roots

Based on the activity of the various FAD2 TAL effector endonucleases in yeast, four TAL effector endonucleases in two different scaffolds were chosen for expression in soybean cells: FAD2_T01 in the C40 architecture, FAD2_T02 in the C40 architecture, FAD2_T03, in the C40 architecture, and FAD2_ T04 in the C11 architecture. The activities of these TAL effector endonucleases were assessed at their endogenous target sites in soybean using a hairy root assay described elsewhere (Curtin et al. Plant Physiology 156(2):466-473, 2011). First, each TAL effector endonuclease was cloned into a T-DNA vector downstream of an estradiol-inducible promoter (Zuo et al., Plant J. 24:265-273, 2000), and then transformed into the K599 strain of *Agrobacterium rhizogenes* (Govindarajulu et al., Mol. Plant Microbe Interact. 21:1027-1035, 2008). *A. rhizogenes* strains with the various TAL effector endonucleases were then used to infect half-cotyledons of soybean and produce transgenic hairy roots. Three weeks after infection, hairy roots were collected and frozen in liquid nitrogen, and genomic DNA was prepared using standard methods (Murray et al., Nucl. Acids Res. 8(19):4321-4325, 1980).

To determine if NHEJ-mediated mutations were created by the TAL effector endonucleases at the target sites in the soybean genome, DNA from nine hairy roots were subjected to a PCR enrichment assay (Zhang et al., Proc. Natl. Acad. Sci. USA 107(26):12028-12033, 2010). This assay monitors loss of a restriction enzyme site within the TAL effector endonuclease spacer sequence due to NHEJ-induced mutations. The restriction enzyme sites used in this assay are shown in FIG. 1 (signified by lower case letters). Genomic DNA derived from three hairy root samples expressing FAD2_T01 was digested with Mn1I to monitor mutations at the FAD2-1A locus. HphI was used to monitor mutations at FAD2-1B caused by FAD2_T01. AciI was used to monitor mutations at both the FAD2-1A and FAD2-1B loci in digested genomic DNA from six hairy root samples expressing FAD2_T04. Following digestion, an aliquot of each reaction was used as a template for PCR with primers flanking either the FAD2_T01 or FAD2_T04 target sites. To assess activity of FAD2_T01, separate PCR reactions were conducted with primers specific for FAD2-1A and FAD2-1B. PCR products were first analyzed on a 1% agarose gel to verify that amplification had occurred, and then digested with the restriction enzyme located in the spacer sequence of the particular TAL effector endonuclease being analyzed. This second restriction digestion reaction was then analyzed on a 2% agarose gel, along with digested and undigested control DNA from wild type samples. Samples with TAL effector endonuclease-induced NHEJ mutations may lack the restriction enzyme site within the spacer sequence, resulting in an undigested PCR product which appears as a full-length band on the gel. As shown in FIG. 2, undigested PCR products were observed for FAD2-1A, FAD2-1B, or both genes in the same sample. For example, sample "b" in FIG. 2C shows that mutations were present in FAD2-1B, sample "d" in FIG. 2B shows that mutations were present in FAD2-1A, and sample "f" in FIGS. 2B and 2D shows that mutations were present in both FAD2-1A and FAD2-1B.

Undigested PCR products were cloned and sequenced to verify that they contained TAL effector endonuclease-induced mutations in the spacer sequence. The PCR products were cloned using the Qiagen TOPO cloning kit according to manufacturer's instructions. Individual clones derived from a given undigested fragment were sequenced, and the DNA sequences aligned with the wild type FAD2-1A or FAD2-1B sequences. As shown in FIG. 3, multiple, independent deletions were recovered in both FAD2-1A and FAD2-1B, ranging in size from 6 to 92 base pairs.

### Example 4 - Regeneration of soybean plants with TAL effector endonuclease-induced mutations in FAD2 genes

Following verification that FAD2_TAL01 and FAD2_TAL04 created targeted modifications at endogenous target sites, experiments were conducted to create soybean plants with mutations in FAD2-1A and FAD2-1B. To accomplish this, each of the four FAD2-1 TAL effector endonucleases were cloned into a T-DNA vector, and TAL effector endonuclease expression was driven by either the cauliflower mosaic virus 35S promoter or the estradiol-induced XVE promoter system (Zuo et al., *supra).* The T-DNA vector also contained a bar selectable marker that confers resistance to glufosinate.

Transgenic soybean plants expressing the TAL effector endonucleases were generated using standard *Agrobacterium tumefaciens* transformation protocols (Curtin et al., *supra).* Following cultivation of the T-DNA-containing *A. tumefaciens* strains with soybean half cotyledons (variety Bert), putatively transgenic plants were regenerated. The plants were transferred to soil, and after approximately 4 weeks of growth, a small leaf was harvested from each plant for DNA extraction and genotyping. Each DNA sample was first screened using PCR for the presence of the T-DNA. All T-DNA-positive plants were then subjected to a T7E1 assay to identify plants with mutations at the FAD2-1A and FAD2-1B TAL effector endonuclease recognition sites (Kim et al., Genome Res. 19:1279-1288, 2009). Briefly, a PCR product spanning the TAL effector endonuclease recognition site was generated, denatured, and allowed to reanneal. T7E1 endonuclease was added to the annealed products to cleave heteroduplexes generated when a wild type DNA fragment annealed with a fragment carrying a TAL effector endonuclease-induced mutation, and cleavage products were visualized by agarose gel electrophoresis. As shown in FIG. 4, four plants showed evidence of TAL effector endonuclease-induced mutations (GM026-18, GM026-23, GM027-06 and GM027-07). In addition, all four plants had mutations at both FAD2-1A and FAD2-1B, indicating that both genes were mutagenized simultaneously. The genotyping data for all plants regenerated is shown in Table 4B.

To determine if mutations introduced by TAL effector endonucleases in leaf tissue were transmitted to the next generation, seeds were collected from M0 plants GM026-18, GM026-23 and GM027-06. In each M1 population, 20-60 individual plants were genotyped to confirm transmission of the mutations. Both FAD2-1A and FAD2-1B mutations segregated in the M1 progeny of GM026-18. In contrast, only FAD2-1A or FAD2-1B mutations were transmitted to the M1 progeny of GM-026-23 and GM027-6, respectively. The DNA sequences of the heritable mutations are shown in FIG. 5.

### Example 5 - Fatty acid profile analysis on seeds produced from TAL effector endonuclease-induced mutations in soybean FAD2 genes

Soybean line GM026-18 was grown to maturity and allowed to self-fertilize, giving rise to seeds that were homozygous mutant in either FAD2-1A (designated aaBB) or FAD2-1B (designated AAbb), or homozygous for both FAD2-1A and FAD2-1B (designated aabb). These seeds were analyzed for fatty acid composition. Briefly, individual soybean seeds were pulverized, and DNA was prepared from a portion of the ground tissue and was analyzed to establish the genotype of each seed. The remaining pulverized tissue from FAD2-1A homozygous (AAbb), FAD2-1B homozygous (aaBB), or FAD2-1A/FAD2-1B double homozygous (aabb) knock out seeds was used to determine fatty acid composition using fatty acid methyl esters (FAME) gas chromatography (Beuselinck et al., Crop Sci. 47:747-750, 2006). The results of the analysis are shown in FIG. 6. As can be seen in the figure, the wild type plants and the aaBB and AAbb lines showed similar fatty acid profiles - approximately 20% oleic acid and 50% linoleic acid. However, the aabb line that was homozygous mutant for FAD2-1A and FAD2-1B showed highly elevated levels of oleic acid that approximated 80%. The levels of linoleic acid in the aabb line were greatly reduced, and were less than 3%.

**Table 4A**

| *List of regenerated plants and FAD2-1 genes analyzed in* *FIG. 4* | | |
|---|---|---|
| **Lane** | **DNA** | **Gene** |
| 1 | GM026-7a | FADII 1A |
| 2 | GM026-17 | FADII 1A |
| 3 | GM026-18 | FADII 1A |
| 4 | GM026-20 | FADII 1A |
| 5 | GM026-23 | FADII 1A |
| 6 | GM027-3 | FADII 1A |
| 7 | GM027-6 | FADII 1A |
| 8 | GM027-7 | FADII 1A |
| 9 | GM027-10 | FADII 1A |
| 10 | GM027-11 | FADII 1A |
| 11 | GM008-6 | FADII 1A |
| 12 | Bert WT | FADII 1A |
| 13 | GM026-7a | FADII 1A uncut |
| 14 | GM026-17 | FADII 1A uncut |
| 15 | GM026-18 | FADII 1A uncut |
| 16 | GM026-7a | FADII 1B |
| 17 | GM026-17 | FADII 1B |
| 18 | GM026-18 | FADII 1B |
| 19 | GM026-20 | FADII 1B |
| 20 | GM026-23 | FADII 1B |
| 21 | GM027-3 | FADII 1B |
| 22 | GM027-6 | FADII 1B |
| 23 | GM027-7 | FADII 1B |
| 24 | GM027-10 | FADII 1B |
| 25 | GM027-11 | FADII 1B |
| 26 | GM008-6 | FADII 1B |
| 27 | Bert WT | FADII 1B |
| 28 | GM026-7a | FADII 1B uncut |
| 29 | GM026-17 | FADII 1B uncut |
| 30 | GM026-18 | FADII 1B uncut |

**Table 4B**

| *Mutant Screening in T0 Transgenic Soybean Plants* | | | | | | | | | | |
|---|---|---|---|---|---|---|---|---|---|---|
| | **GM026- 7a** | **GM026- 17** | **GM026- 18** | **GM026- 20** | **GM026- 23** | **GM027- 3** | **GM027- 6** | **GM027- 7** | **GM027- 10** | **GM027-11** |
| **FAD2-1A** | Wt | Wt | Mutation | Wt | Mutation | Wt | Mutation | Mutation | Wt | Wt |
| **FAD2-1B** | Wt | Wt | Mutation | Wt | Mutation | Wt | Mutation | Mutation | Wt | Wt |

| | | | | | | | | | | |
|---|---|---|---|---|---|---|---|---|---|---|
| *Wt signifies wild type genotype based on T7E1 assay | | | | | | | | | | |

**Table 5**

| *Representative FAD2-1A and FAD2-1B coding sequences** |
|---|
| FAD2-1A |
| |
| FAD2-1B |
| |
| |

| |
|---|
| *intron sequences are in lower case |

### Example 6 - Generation of transgene-free mutant soybean lines

To obtain soybean lines that had the FAD2-1 mutations but lacked the FAD2_T04 TAL effector endonuclease construct and its associated selectable marker, progeny of GM026-18 were screened for the aabb genotype. Mutant, transgene-free segregants were then sought using a PCR strategy that utilized two sets of primer pairs: one spanning the TAL effector endonuclease coding sequence and the other the endogenous alcohol dehydrogenase gene of soybean (a control for the PCR reaction; FIG. 7). Fifteen aabb progeny were analyzed, and six showed no evidence of TALEN sequences in their genome, suggesting the TAL effector endonuclease construct had segregated away in these plants. These results indicate the feasibility of creating transgene-free lines with mutations in both the FAD2-1A and FAD2-1B genes.

## Claims

1. A method for producing soybean oil having increased oleic acid content and reduced linoleic acid content, comprising:
(a) providing a soybean plant or plant part comprising:
(i) a deletion in one or more FAD2-1A alleles, wherein said deletion in the one or more FAD2-1A alleles was induced by TAL effector endonucleases binding to SEQ ID NOs: 32 and 33, and
(ii) a deletion in one or more FAD2-1B alleles, wherein said deletion in the one or more FAD2-1B alleles was induced by TAL effector endonucleases binding to SEQ ID NOs: 32 and 33; and
(b) producing oil from seed of said plant or plant part, wherein said oil has increased oleic acid content and reduced linoleic acid content as compared to oil produced by a soybean plant or plant part that does not comprise said deletions.

2. The method of claim 1, wherein said plant or plant part does not contain a transgene.

3. The method of claim 1, wherein said one or more FAD2-1B alleles comprise SEQ ID NO: 16.

4. A method for making a soybean plant comprising a deletion in each FAD2-1A allele and a deletion in each FAD2-1B allele, said method comprising:
(a) contacting a population of soybean plant cells comprising functional FAD2-1A and FAD2-1B alleles with TAL effector endonucleases targeted to SEQ ID NOs: 32 and 33,
(b) selecting, from said population, a cell in which each FAD2-1A allele and each FAD2-1B allele comprises a deletion,
wherein said deletion in each FAD2-1A allele was induced by said TAL effector endonucleases targeted to SEQ ID NOs: 32 and 33, and
wherein said deletion in each FAD2-1B allele was induced by said TAL effector endonucleases targeted to SEQ ID NOs: 32 and 33, and
(c) regenerating said selected plant cell into a soybean plant.

5. The method of claim 4, wherein said soybean plant cells comprise cotyledon cells.

6. The method of claim 5, wherein said method comprises transforming said cotyledon cells with one or more vectors encoding said TAL effector endonucleases.

7. The method of claim 4, further comprising culturing said plant cells to generate plant lines.

8. The method of claim 4, wherein each FAD2-1B allele in said selected cell comprises SEQ ID NO: 16.

## Patentansprüche

1. Verfahren zur Herstellung von Sojaöl mit erhöhtem Ölsäuregehalt und reduziertem Linolsäuregehalt, umfassend:
(a) Bereitstellen einer Sojabohnenpflanze bzw. einem Sojabohnenpflanzenteil, die bzw. der Folgendes umfasst:
(i) eine Deletion in einem oder mehreren FAD2-1A-Allelen, wobei die Deletion in dem einen bzw. den mehreren FAD2-1A-Allelen durch an SEQ ID NO: 32 und 33 bindende TAL-Effektor-Endonukleasen induziert wurde, und
(ii) eine Deletion in einem oder mehreren FAD2-1B-Allelen, wobei die Deletion in dem einen bzw. den mehreren FAD2-1B-Allelen durch an SEQ ID NO: 32 und 33 bindende TAL-Effektor-Endonukleasen induziert wurde; und
(b) Herstellen von Öl aus Samen der Pflanze bzw. des Pflanzenteils, wobei das Öl einen erhöhten Ölsäuregehalt und reduzierten Linolsäuregehalt im Vergleich zu Öl, das von einer Sojabohnenpflanze bzw. einem Sojabohnenpflanzenteil, die bzw. der die Deletionen nicht umfasst, produziert wird, aufweist.

2. Verfahren nach Anspruch 1, wobei die Pflanze bzw. der Pflanzenteil kein Transgen enthält.

3. Verfahren nach Anspruch 1, wobei die ein oder mehreren FAD2-1B-Allele SEQ ID NO: 16 umfassen.

4. Verfahren zur Erzeugung einer Sojabohnenpflanze, die eine Deletion in jedem FAD2-1A-Allel und eine Deletion in jedem FAD2-1B-Allel umfasst, wobei das Verfahren Folgendes umfasst:
(a) In-Kontakt-Bringen einer Population von funktionsfähige FAD2-1A- und FAD2-1B-Allele umfassenden Sojabohnenpflanzenzellen mit auf SEQ ID NO: 32 und 33 gerichteten TAL-Effektor-Endonukleasen,
(b) Selektieren einer Zelle, in der jedes FAD2-1A-Allel und jedes FAD2-1B-Allel eine Deletion umfasst, aus der Population,
wobei die Deletion in den einzelnen FAD2-1A-Allelen jeweils durch die auf SEQ ID NO: 32 und 33 gerichteten TAL-Effektor-Endonukleasen induziert wurde und
wobei die Deletion in den einzelnen FAD2-1B-Allelen jeweils durch die auf SEQ ID NO: 32 und 33 gerichteten TAL-Effektor-Endonukleasen induziert wurde, und
(c) Regenerieren der selektierten Pflanzenzelle zu einer Sojabohnenpflanze.

5. Verfahren nach Anspruch 4, wobei die Sojabohnenpflanzenzellen Keimblattzellen umfassen.

6. Verfahren nach Anspruch 5, wobei das Verfahren Transformieren der Keimblattzellen mit einem oder mehreren Vektoren, die die TAL-Effektor-Endonukleasen codieren, umfasst.

7. Verfahren nach Anspruch 4, ferner umfassend Kultivieren der Pflanzenzellen zur Erstellung von Pflanzenlinien.

8. Verfahren nach Anspruch 4, wobei die FAD2-1B-Allele in der selektierten Zelle jeweils SEQ ID NO: 16 umfassen.

## Revendications

1. Procédé de production d'une huile de soja ayant une teneur accrue en acide oléique et une teneur réduite en acide linoléique, comprenant :
(a) la fourniture d'une plante ou d'une partie de plante de soja, comprenant :
(i) une délétion dans un ou plusieurs allèles FAD2-1A, ladite délétion dans un ou plusieurs allèles FAD2-1A ayant été induite par des effecteur TAL endonucléases se liant aux SEQ ID NO : 32 et 33, et
(ii) une délétion dans un ou plusieurs allèles FAD2-1B, ladite délétion dans un ou plusieurs allèles FAD2-1B ayant été induite par des effecteur TAL endonucléases se liant aux SEQ ID NO : 32 et 33 ; et
(b) la production d'huile à partir de graines de ladite plante ou de ladite partie de plante, ladite huile présentant une teneur accrue en acide oléique et une teneur réduite en acide linoléique par comparaison avec une huile produite par une plante ou une partie de plante de soja qui ne comprend pas lesdites délétions.

2. Procédé selon la revendication 1, dans lequel ladite plante ou ladite partie de plante ne contient pas de transgène.

3. Procédé selon la revendication 1, dans lequel ledit un ou plusieurs allèles FAD2-1B comprennent la SEQ ID NO : 16.

4. Procédé de fabrication d'une plante de soja comprenant une délétion dans chaque allèle FAD2-1A et une délétion dans chaque allèle FAD2-1B, ledit procédé comprenant :
(a) la mise en contact d'une population de cellules de plante de soja comprenant des allèles FAD2-1A et FAD2-1B fonctionnels avec des effecteur TAL endonucléases ciblées vers les SEQ ID NO : 32 et 33,
(b) la sélection, dans cette population, d'une cellule dans laquelle chaque allèle FAD2-1A et chaque allèle FAD2-1B comprend une délétion,
ladite délétion dans chaque allèle FAD2-1A ayant été induite par lesdites effecteur TAL endonucléases ciblées vers les SEQ ID NO : 32 et 33, et
ladite délétion dans chaque allèle FAD2-1B ayant été induite par lesdites effecteur TAL endonucléases ciblées vers les SEQ ID NO : 32 et 33, et
(c) la régénération de ladite cellule de plante sélectionnée pour obtenir une plante de soja.

5. Procédé selon la revendication 4, dans lequel lesdites cellules de plante de soja comprennent des cellules de cotylédon.

6. Procédé selon la revendication 5, ledit procédé comprenant la transformation desdites cellules de cotylédon avec un ou plusieurs vecteurs codant pour lesdites effecteur TAL endonucléases.

7. Procédé selon la revendication 4, comprenant en outre la culture desdites cellules de plante pour générer des lignées de plantes.

8. Procédé selon la revendication 4, dans lequel chaque allèle FAD2-1B de ladite cellule sélectionnée comprend la SEQ ID NO : 16.
